(19)

Europäisches Patentamt
European Patent Office
Office européen des brevets

(11) **EP 3 561 522 A1**

(12) **EUROPEAN PATENT APPLICATION**
published in accordance with Art. 153(4) EPC

(43) Date of publication:
**30.10.2019 Bulletin 2019/44**

(21) Application number: **17882506.3**

(22) Date of filing: **12.12.2017**

(51) Int Cl.:
$G01Q\ 30/04^{(2010.01)}$ $A61B\ 5/00^{(2006.01)}$

(86) International application number:
**PCT/KR2017/014538**

(87) International publication number:
**WO 2018/117516 (28.06.2018 Gazette 2018/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **22.12.2016 KR 20160177072**
**30.08.2017 KR 20170110486**

(71) Applicants:
• **Seoul National University Hospital**
**Seoul 03080 (KR)**
• **Korea University Research and Business Foundation**
**Seoul 02841 (KR)**

(72) Inventors:
• **CHOI, Tae Hyun**
**Seoul 06219 (KR)**
• **JO, Seong Jin**
**Seoul 05502 (KR)**
• **HUR, Woojune**
**Seoul 04304 (KR)**
• **JEON, Byoung Jun**
**Seoul 03067 (KR)**
• **WUFUER, Maierdanjiang**
**Seoul 04988 (KR)**

• **KIM, Youngmin**
**Gimpo-si**
**Gyeonggi-do 10129 (KR)**
• **PARK, Gee Ho**
**Guri-si**
**Gyeonggi-do 11921 (KR)**
• **HUANG, Yan**
**Seoul 03065 (KR)**
• **KIM, Jungah**
**Ansan-si**
**Gyeonggi-do 15290 (KR)**
• **KIM, Byung Jun**
**Seoul 06219 (KR)**
• **SHIM, Jung Hee**
**Seoul 06003 (KR)**
• **CHOI, Eunjung**
**Seoul 04728 (KR)**
• **YOON, Dae Sung**
**Seoul 06359 (KR)**
• **LEE, Wonseok**
**Incheon 21104 (KR)**
• **LEE, Gyudo**
**Gyeonggi-do 12102 (KR)**

(74) Representative: **Botti, Mario**
**Botti & Ferrari S.r.l.**
**Via Cappellini, 11**
**20124 Milano (IT)**

(54) **METHOD AND SYSTEM FOR DIAGNOSING MALIGNANT MELANOMA USING SCANNING PROBE MICROSCOPE**

(57) Disclosed is a method for determining malignant melanoma by a scanning probe microscope system with a cantilever, which includes: setting locations of a plurality of measurement points to be measured in a sample tissue; applying force in a predetermined range to each measurement point on the sample tissue through the cantilever and acquiring information on a distance between a probe and the sample tissue depending on force for each measurement point; generating a force-distance graph of measurement points based on distance information depending on the force acquired at the plurality of measurement points; and determining whether the sample tissue is malignant melanoma based on characteristics information of the sample tissue extracted from the force-distance graph.

# FIG. 1

Tissue biopsy    Paraffin embedding    Block section and staining

slide storage    AFM indentation    Cantilever

(a)

Sample tissue

Melanoma

Epidermis

Melanin cell

Dermis

(b)

**Description**

**[Technical Field]**

The present disclosure relates to a malignant melanoma determination technique.

**[Background Art]**

The number of people who die from cancer in Korea is 30% or more of the total deaths annually and there is a trend that skin cancer continuously increases in occurrence frequency due to an increase in exposure opportunity of skin to ultraviolet rays and various harmful substances due to environmental pollution. Skin cancer may be various as squamous cell carcinoma, basal cell carcinoma, and malignant melanoma, and among them, malignant melanoma as a malignant tumor of melanocyte may occur in any part where melanocyte is present and is very dangerous cancer with low 5-year survival rate because malignant melanoma is often found in a late stage.

Until now, clinical diagnostic techniques of general skin cancer and malignant melanoma have mainly adopted an ABCD discrimination method which distinguishes features such as lesion asymmetry, border, color, and diameter through the eye of a doctor and judgment method through genetic family history. Approximately 56% to 90% of skin cancer diagnoses go through the ABCD discrimination method and a histopathological examination procedure and have sensitivity of approximately 60%. Therefore, it is difficult to distinguish nevus and malignant melanoma from each other when the lesion of a patient is first checked.

Optical Dermoscopy techniques have been used to detect malignant melanoma lesions through a polarized light source to overcome the limitations of existing pathological diagnostic techniques and to diagnose malignant melanoma. However, this technique can only be used and interpreted by trained or clinically experienced specialists and has low sensitivity.

Recently, development of various cancer-related cell and tissue analysis technologies has progressed in accordance with the development of nano-micro technology at home and abroad, and there are studies of melanoma at the cellular level, but cases in the tissues are rare. Among existing nanotechnologies, optical and magnetic tweezing technologies require separate MEMS/NEMS processes and complex experimental methods, and there is a limit that the characteristics of malignant melanoma cannot be completely explained. In particular, there is no technology that can distinguish clear differences from nevus tissues in which misdiagnosis may occur as well as detecting malignant melanoma, and accurate identification technology is therefore required.

Therefore, in current skin cancer and malignant melanoma diagnosis techniques, which have low sensitivity and possibility of misdiagnosis, it is necessary to quickly distinguish malignant melanoma clearly and to develop quantitative malignant melanoma diagnosis technology.

**[DISCLOSURE]**

**[Technical Problem]**

The present disclosure provides a method and a system for acquiring mechanical characteristics including a force-distance graph of skin tissue through a scanning probe microscope and determining malignant melanoma based thereon.

**[Technical Solution]**

An exemplary embodiment of the present disclosure provides a method for determining malignant melanoma by a scanning probe microscope system with a cantilever, which includes: setting locations of a plurality of measurement points to be measured in a sample tissue; applying force in a predetermined range to each measurement point on the sample tissue through the cantilever and acquiring information on a distance between a probe and the sample tissue depending on force for each measurement point; generating a force-distance graph of measurement points based on distance information depending on the force acquired at the plurality of measurement points; and determining whether the sample tissue is malignant melanoma based on characteristics information of the sample tissue extracted from the force-distance graph.

The characteristics information may include at least one of linearity of the force-distance graph, a slope of the force-distance graph, stiffness distribution of the plurality of measurement points, and a stiffness distribution probability of the plurality of measurement points.

The method may further include generating the stiffness distribution probability graph of the sample tissue based on the force-distance graph and in the determining of whether the sample tissue is the malignant melanoma, when the force-distance graph has non-linearity and the stiffness distribution probability graph has multi-peaks, the sample tissue may be determined as the malignant melanoma.

In the determining of whether the sample tissue is the malignant melanoma, when the force-distance graph has linearity and the stiffness distribution probability graph has a single peak, the sample tissue may be determined as normal tissue.

In the determining of whether the sample tissue is the malignant melanoma, when the force-distance graph has non-linearity and the stiffness distribution probability graph has the single peak, the sample tissue may be determined as nevus tissue.

In the determining of whether the sample tissue is the malignant melanoma, when a first force-distance graph of the sample tissue is different from a second force-distance graph of the normal tissue and the stiffness distribution probability graph of the sample tissue derived from the first force-distance graph has a plurality of peaks, the sample tissue may be determined as the malignant melanoma.

In the determining of whether the sample tissue is the malignant melanoma, when the first force-distance graph is different from the second force-distance graph and the stiffness distribution probability graph of the sample tissue derived from the first force-distance graph has the single peak, the sample tissue may be determined as the nevus tissue.

Another exemplary embodiment of the present disclosure provides a scanning probe microscope system which includes: a cantilever with a probe; and a controller applying force in a predetermined range to each of a plurality of measurement points on a sample tissue through the cantilever, generating a force-distance graph of measurement points based on distance information between the probe and the sample tissue depending on force for each measurement point, and determining whether the sample tissue is malignant melanoma based on characteristics information of the sample tissue extracted from the force-distance graph.

The characteristics information may include at least one of linearity of the force-distance graph, a slope of the force-distance graph, stiffness distribution of the plurality of measurement points, and a stiffness distribution probability of the plurality of measurement points.

The controller may generate a stiffness distribution probability graph of the sample tissue based on the force-distance graph and determine the sample tissue as the malignant melanoma when the force-distance graph has non-linearity and the stiffness distribution probability graph has multi-peaks.

The controller may determine, when the force-distance graph has linearity and the stiffness distribution probability graph has a single peak, the sample tissue as normal tissue, and determine, when the force-distance graph has non-linearity and the stiffness distribution probability graph has the single peak, the sample tissue as nevus tissue.

The controller may determine, when a first force-distance graph of the sample tissue is different from a second force-distance graph of the normal tissue and the stiffness distribution probability graph of the sample tissue derived from the first force-distance graph has a plurality of peaks, the sample tissue as the malignant melanoma.

The controller may determine, when the first force-distance graph is different from the second force-distance graph and the stiffness distribution probability graph of the sample tissue derived from the first force-distance graph has the single peak, the sample tissue as the nevus tissue.

The cantilever may have a resonance frequency of 204 to 497 KHz and a spring constant of 10 to 130 N/m.

The sample tissue may be skin tissue including epidermis and dermis.

[Advantageous Effects]

According to an exemplary embodiment of the present disclosure, it is possible to reduce a low sensitivity and a possibility of misdiagnosis of the existing malignant melanoma detection technique, and to diagnose malignant melanoma early. In particular, according to an exemplary embodiment of the present disclosure, malignant melanoma can be rapidly and clearly distinguished by acquiring tissue characteristics of malignant melanoma different from normal or nevus tissue due to abnormal melanocyte differentiation, and quantified malignant melanoma diagnostic indicators can be provided.

Compared to the related art which can be used and interpreted only by a specialist having a great deal of clinical experience, according to an exemplary embodiment of the present disclosure, nevus and malignant melanoma can be distinguished due to mechanical characteristics that malignant melanoma tissue staining sample containing a biotissue is measured with a scanning probe microscope.

[Description of the Drawings]

FIG. 1 is a diagram for describing acquisition and mounting of a sample tissue for a scanning probe microscope according to an exemplary embodiment of the present disclosure.

FIG. 2 is a configuration diagram of a scanning probe microscope system according to an exemplary embodiment of the present disclosure.

FIG. 3 illustrates examples of surface images of normality, nevus, and malignant melanoma obtained according to an exemplary embodiment of the present disclosure.

FIG. 4 illustrates an example of a force-distance graph of normal tissue, nevus issue, and malignant melanoma tissue according to an exemplary embodiment of the present disclosure.

FIG. 5 illustrates an example of a stiffness distribution of normal tissue, nevus issue, and malignant melanoma tissue according to an exemplary embodiment of the present disclosure.

FIG. 6 illustrates an example of a stiffness distribution probability graph of normal tissue, nevus issue, and malignant melanoma tissue according to an exemplary embodiment of the present disclosure.

FIG. 7 is a flowchart of a method for diagnosing a tissue according to an exemplary embodiment of the present disclosure.

## [Mode for Invention]

In the following detailed description, only certain exemplary embodiments of the present disclosure have been shown and described, simply by way of illustration. As those skilled in the art would realize, the described embodiments may be modified in various different ways, all without departing from the spirit or scope of the present disclosure. Accordingly, the drawings and description are to be regarded as illustrative in nature and not restrictive. Like reference numerals designate like elements throughout the specification.

Throughout the specification, unless explicitly described to the contrary, the word "comprise" and variations such as "comprises" or "comprising", will be understood to imply the inclusion of stated elements but not the exclusion of any other elements. In addition, the terms "-er", "-or" and "module" described in the specification mean units for processing at least one function and operation and can be implemented by hardware components or software components and combinations thereof.

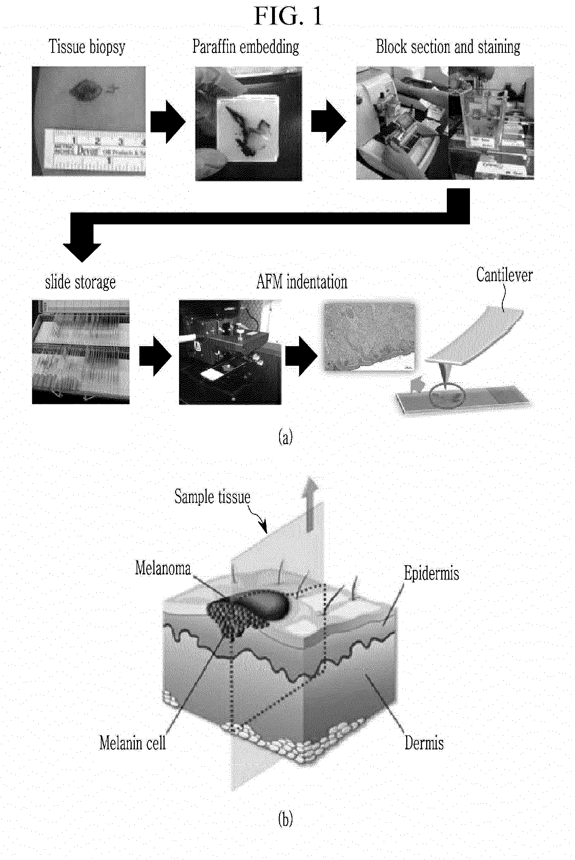

FIG. 1 is a diagram for describing acquisition and mounting of a sample tissue for a scanning probe microscope according to an exemplary embodiment of the present disclosure.

Referring to FIG. 1(a), a skin sample tissue mounted on a scanning probe microscope may be acquired from tissue biopsy as follows.

Paraffin blocks are acquired by paraffin-embedding paraffin used for pathological examination into tissues. Then, the paraffin block is again cut and produced into 4-μm-thick sections.

Thereafter, the tissue sections are then subjected to general Hematoxylin & Eosin staining (H & E Staining) to obtain sample tissues. The sample tissues are made in a form in which the sample tissues may be stored (slide storage). In this case, the sample tissue may be produced as a raw tissue without being stained.

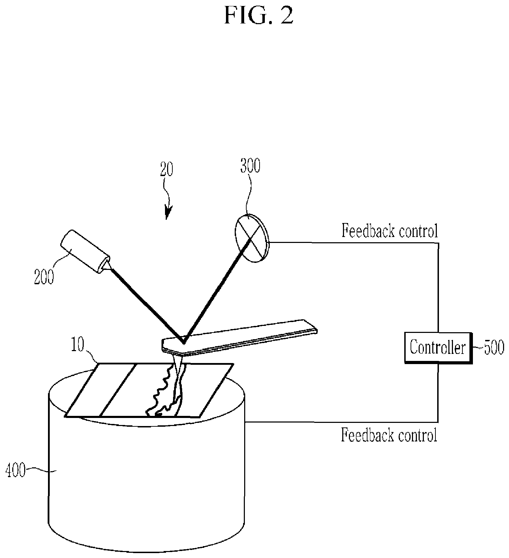

A sample tissue 10 is mounted on a scanning probe microscope system 20 and is pushed by a probe of a cantilever and mechanical characteristics of the sample tissue 10 are obtained by indentation. The scanning probe microscope system 20 including the cantilever will be described in detail in FIG. 2. The mechanical characteristics include a force-distance graph showing a distance pushed according to a force applied at each point of the sample tissue, linearity and slope thereof, and a stiffness index obtained according to the applied force at each point.

Referring to FIG. 1(b), the sample tissue 10 is produced to include the epidermis and the dermis in the skin tissue and, if necessary, a subcutaneous fat layer.

Malignant melanoma may be determined by characteristics of only a sample tissue suspected as malignant melanoma, or by comparing the characteristics of the sample tissue suspected as malignant melanoma with a contrast group contrasted with the sample tissue, malignant melanoma may be determined. Here, the control group may be at least one of a normal sample tissue or a nevus sample tissue. Therefore, the sample tissue 10 may include the sample tissue suspected as malignant melanoma and the normal sample tissue or the nevus sample tissue contrasted thereto.

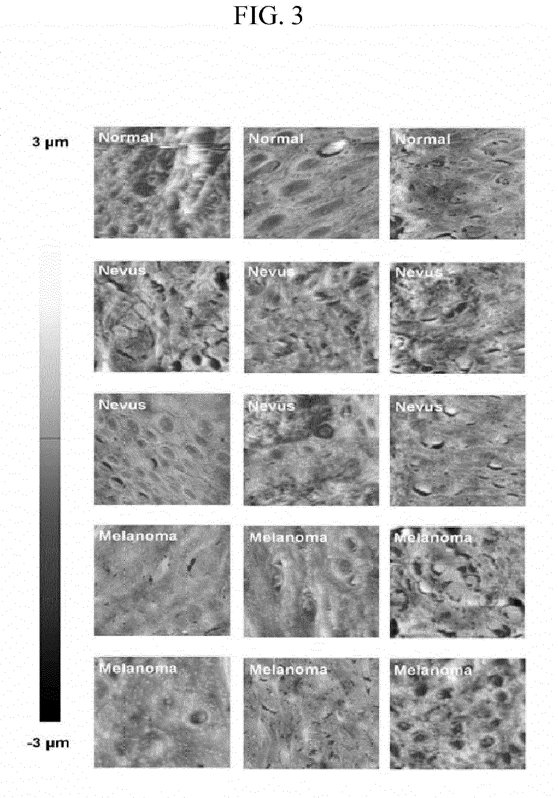

FIG. 2 is a configuration diagram of a scanning probe microscope system according to an exemplary embodiment of the present disclosure and FIG. 3 illustrates examples of surface images of normality, nevus, and malignant melanoma obtained according to an exemplary embodiment of the present disclosure.

Referring to FIG. 2, the scanning probe microscope system 20 acquires mechanical characteristics (force-distance graph and stiffness index) and surface characteristics (surface image) by applying force to the skin sample tissue 10. The scanning probe microscope system 20 may be an atomic force microscope (AFM) which extracts the surface image by pressing the sample tissue 10 with the cantilever or with a vertical bending degree of the cantilever by the force between a probe and atoms of the sample tissue 10.

The scanning probe microscope system 20 includes a cantilever 100 with the probe, a laser 200, a photo detector 300, a piezo scanner 400 for moving the sample tissue 10, and a controller 500. The photo detector 300 may be a photodiode. In the present disclosure, for description, it is assumed that the cantilever 100 is fixed and the sample tissue 10 is moved by the piezo scanner 400, but it may be implemented that the skin sample tissue 10 is fixed and the cantilever 100 moves. In the present disclosure, for description, it is described that the controller 500 determines malignant melanoma while feedback-controlling devices included in the scanning probe microscope system 20, but a determination device that determines malignant melanoma based on information measured from the scanning probe microscope system 20 may be separately implemented.

The scanning probe microscope system 20 includes the cantilever 100, the laser 200, and the photo detector 300 arranged to reflect light emitted from the laser 200 on an upper surface of the cantilever 100 and detect reflection light reflected on the upper surface of the cantilever 100 by the photo detector 300. In addition, the scanning probe microscope system 20 includes the piezo scanner 400 mounted with the sample tissue 10 and horizontally moving the sample tissue 10 or vertically moving the sample tissue 10. The scanning probe microscope system 20 repeats an operation of applying the force to each point while moving to measurement points when locations (e.g., lesion locations) of measurement points or the number of measurement points on a sample tissue from which the mechanical characteristics are to be extracted. For example, an interval between respective measurement points may be within 4.5 μm and approximately 100 points may be set as the measurement points, but the interval and the number of measurement points may be varied according to the sample tissue or a measurement method.

The controller 500 performs feedback control on the photo detector 300 and the piezo scanner 400 and extracts the mechanical characteristics (force-distance graph) and the surface characteristics (surface image) of the skin sample tissue 10 based on information acquired from the photo detector 300 and the piezo scanner 400, and finally determines malignant melanoma.

The probe, the length, the thickness, a material, a resonance frequency, and a spring constant, and the like of the cantilever 100 may be determined according to characteristics and a sample size of skin tissue to be measured. For example, the cantilever 100 may be coated with aluminum with approximately 30 nm, the length may be approximately 115 to 135 $\mu$m, the thickness may be 3 to 5 $\mu$m, the resonance frequency may be 204 to 497 KHz, and the spring constant may be 10 to 130 N/m. Since the cantilever 100 is used for achieving a surface shape image of the sample tissue and needs to press the sample tissue in order to acquire the force-distance graph and the stiffness index, by such points, the probe, the length, the thickness, the material, the resonance frequency, the spring constant, and the like may be determined.

The cantilever 100 is bent vertically by the force between the probe attached to the tip of the cantilever 100 and the atoms of the sample tissue 10 and in this case, the cantilever 100 is bent by an interaction force (Van der Waals force) between the atoms of the probe and the atoms of the sample tissue through the photo detector 300.

The controller 500 generates an image showing a surface shape of the sample tissue 10 by moving the cantilever 100 or the piezo scanner 400 while performing feedback control so that the measured force between the atoms is constantly maintained. In this case, the controller 500 may make geometric surface characteristics of the lesion of the sample tissue by using a tapping mode in which the probe taps the sample tissue 10 into the image. An image size may be various as 60 x 60 to 90 to 90 $\mu$m$^2$.

Referring to FIG. 3, the scanning probe microscope system 20 may acquire surface images of the normal tissue, the nevus tissue, and malignant melanoma in the tapping mode.

The controller 500 generates a distance between the probe and the issue according to the force as the force-distance graph while applying the force to each measurement point of the sample issue 10 through the cantilever 100. The distance between the probe and the tissue is 0 $\mu$m (tissue surface) when the force is 0 $\mu$N, and the probe invades the tissue as the magnitude of the force increases, and as a result, a distance value (e.g., 0 to -0.05 $\mu$m, -0.1 $\mu$m, etc.) may be represented as a negative value. The controller 500 generates the force-distance graph of the measurement points based on distance information depending on force acquired at measurement points (e.g., 100 points at an interval of 4.5 $\mu$m). When the force-distance graph of the measurement points has non-linearity, the sample tissue is determined as malignant melanoma.

The controller 500 generates a stiffness distribution probability graph based on distance information (force-distance graph) according to the force acquired at the measurement points. When the stiffness distribution probability graph has multi-peaks, the sample tissue is determined as malignant melanoma.

When the force-distance graph has the non-linearity and the stiffness distribution probability graph has multi-peaks, the controller 500 determines the sample tissue as malignant melanoma in order to increase accuracy. Alternatively, when the force-distance graph has the non-linearity equal to or more than a reference value, the controller 500 may determine the sample tissue as malignant melanoma and when stiffness distribution probability graph has multi-peaks, the controller 500 may determine the sample tissue as malignant melanoma.

When the force-distance graph has linearity and the stiffness distribution probability graph has a single peak, the controller 500 determines the sample tissue as the normal tissue. When the force-distance graph has pseudo-linearity or non-linearity and the stiffness distribution probability graph has the single peak, the controller 500 determines the sample tissue as the nevus tissue.

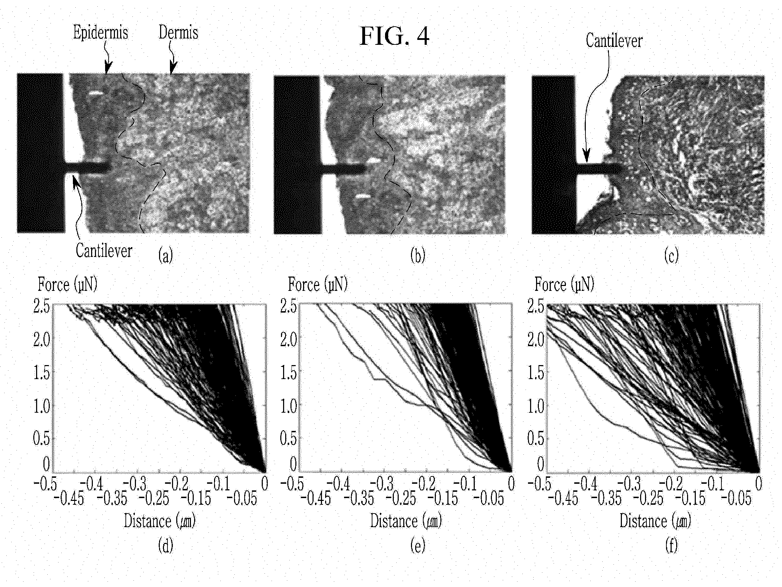

FIG. 4 illustrates an example of a force-distance graph of normal tissue, nevus tissue, and malignant melanoma tissue according to an exemplary embodiment of the present disclosure.

Referring to FIG. 4, FIG. 4(a) illustrates an optical image acquired by photographing the cantilever positioned on the normal tissue. FIG. 4(b) illustrates an optical image acquired by photographing the cantilever positioned on the nevus tissue. FIG. 4(c) illustrates an optical image acquired by photographing the cantilever positioned on the malignant melanoma tissue.

As such, a force-distance graph acquired by applying the force to the cantilever at measurement points including the normal tissue, the nevus tissue, and the malignant melanoma tissue is shown in (d), (e), and (f). The linearity and the slope of the force-distance graph acquired at the measurement points show characteristics of each sample tissue. A linear graph means viscosity of the tissue decreases. The higher slope, the stiffer the tissue.

Referring to the force-distance graph (d) of the normal tissue, the graph acquired at the measurement points is linear.

Referring to the force-distance graph (f) of the malignant melanoma, the graph acquired at the measurement points is non-linear and a slope distribution is also various. That is, it can be seen that the malignant melanoma tissue is shown as a non-linear force-distance graph.

Referring to the force-distance graph (e) of the nevus tissue, the graph acquired at the measurement points is substantially linear and is higher in slope than the force-distance graph (d) of the normal tissue. Accordingly, it can be seen that the nevus tissue is stiffer than the normal tissue.

As described above, since the malignant melanoma tissue clearly shows a difference in mechanical characteristics from the normal tissue, and the difference in characteristics from the nevus tissue, which was difficult to detect in the method in the related art, is clearly distinguished, the scanning probe microscope system 20 may determine, based on the force-distance graph obtained from a predetermined sample tissue, whether the sample tissue is malignant melanoma.

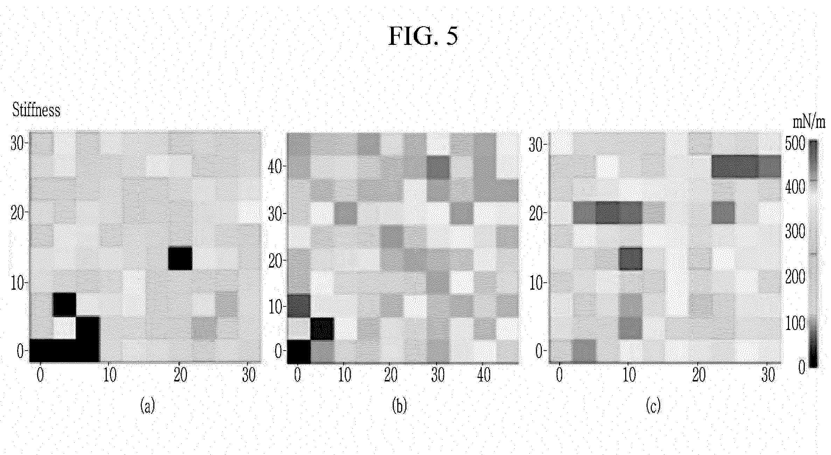

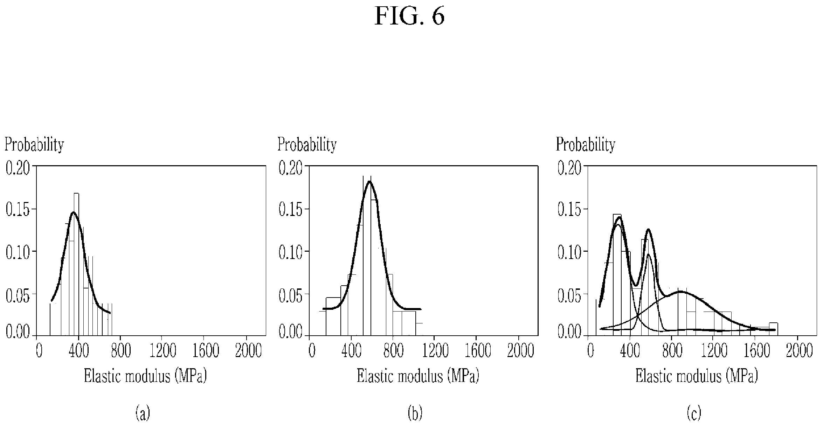

FIG. 5 illustrates an example of a stiffness distribution of normal tissue, nevus issue, and malignant melanoma tissue according to an exemplary embodiment of the present disclosure and FIG. 6 illustrates an example of a stiffness distribution probability graph of normal tissue, nevus issue, and malignant melanoma tissue according to an exemplary embodiment of the present disclosure.

Referring to FIG. 5, when the stiffness distribution of the measurement points acquired from the force-distance graphs (d), (e), and (f) of FIG. 4 is represented by a color, there is a distribution difference depending on the normal tissue, the nevus tissue, and the malignant melanoma tissue. That is, in the acquired force-distance graph, the stiffness distribution may be represented by the color as illustrated in FIG. 5 and an index indicating stiffness may also be derived by using a Hertz model. In FIG. 5, a mesh block is a diagram in which a stiffness distribution of 100 (= 10 x 10) points is represented by the color.

According to the stiffness distribution, the stiffness distributions of the normal tissue (a) and the nevus tissue (b) show uniform characteristics and the nevus tissue (b) has more stiffness tissue distributions than the normal tissue (a). Meanwhile, it can be seen that in the malignant melanoma tissue (c), the stiffness distribution is dispersed from soft tissue to stiff tissue.

Accordingly, based on a stiffness distribution acquired in a predetermined sample tissue, it may be distinguished whether the sample tissue is malignant melanoma.

Referring to FIG. 6, when the stiffness distribution probability graph acquired from the force-distance graphs (d), (e), and (f) of FIG. 4 is represented, there is a difference depending on the normal tissue, the nevus tissue, and the malignant melanoma tissue.

An elastic modulus of FIG. 6 may be acquired by a Hertz model of contact mechanics shown in Equation 1 and acquired by applying the same. In Equation 1, F represents force applied per distance between the probe and the sample, E represents the elastic modulus of the tissue, R represents a diameter of the probe, and δ represents a pushing degree of the sample by the probe.

[Equation 1]

Hertz model :
$$F = \frac{4}{3} E^* \sqrt{R} \, \delta^{3/2}$$

The stiffness distribution probability graphs of the normal tissue (a) and the nevus tissue (b) shows a Gaussian distribution with the single peak. Unlike this, the malignant melanoma tissue (c) shows a Gaussian distribution with multi-peaks. Accordingly, based on the stiffness distribution probability graph acquired in a predetermined sample tissue, it may be distinguished whether the sample tissue is malignant melanoma.

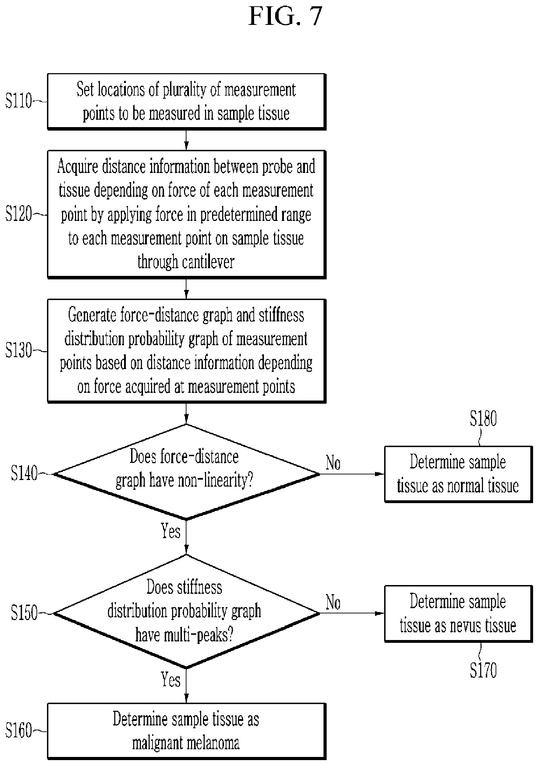

FIG. 7 is a flowchart of a method for diagnosing a tissue according to an exemplary embodiment of the present disclosure.

Referring to FIG. 7, the controller 500 of the scanning probe microscope system 20 sets the locations of a plurality of measurement points to be measured in the sample tissue (S110). The measurement points may be set, for example, as 100 points at an interval of 9 μm.

The controller 500 generates information on a distance between the probe and the issue according to the force at each measurement point while applying force in a predetermined range to each measurement point on the sample issue through the cantilever 100 (S120). The scanning probe microscope system 20 may be an atomic force microscope (AFM) system. The length of the cantilever 100 may be approximately 115 to 135 μm, the thickness may be 3 to 5 μm, the resonance frequency may be 204 to 497 KHz, and the spring constant may be 10 to 130 N/m.

The controller 500 generates the force-distance graph and the sample stiffness distribution probability graph of the measurement points based on distance information depending on force acquired at measurement points (e.g., 100 points at an interval of 9 μm) (S130). Here, the distance may be a distance at which the probe invades a tissue surface.

The controller 500 determines whether the force-distance graph of the measurement points has non-linearity. A criterion for determining the non-linearity and the linearity may be various as a linearity degree of the force-distance graph of each measurement point or the number of linear measurement points.

When the force-distance graph has the non-linearity, the controller 500 determines whether the stiffness distribution probability graph has multi-peaks (S150).

When the stiffness distribution probability graph has multi-peaks, the controller 500 determines the sample tissue as malignant melanoma (S160).

When the stiffness distribution probability graph has the single peak, the controller 500 determines the sample tissue as the nevus tissue (S170).

When the force-distance graph has linearity, the controller 500 determines the sample tissue as the nevus tissue (S180).

Meanwhile, the controller 500 may determine whether the sample tissue is the normal tissue by comparing the slope, the linearity, etc., of the force-distance graph of the sample tissue based on the force-distance graph of the normal tissue. When the sample tissue is not the normal tissue, the controller 500 may distinguish the nevus and malignant melanoma based on the number of peaks of the stiffness distribution probability graph.

The controller 500 may be designed to sequentially determine whether the force-distance graph has the non-linearity and the stiffness distribution probability graph has multi-peaks and determine the normal tissue, the nevus tissue, and the malignant melanoma stepwise. Alternatively, the controller may be designed to determine the normal tissue, the nevus tissue, and the malignant melanoma based on at least one of whether the force-distance graph has the non-linearity and the stiffness distribution probability graph has multi-peaks. As described above, according to an exemplary embodiment of the present disclosure, it is possible to reduce a low sensitivity and a possibility of misdiagnosis of the existing malignant melanoma detection technique, and to diagnose malignant melanoma early. In particular, according to an exemplary embodiment of the present disclosure, malignant melanoma can be rapidly and clearly distinguished by acquiring tissue characteristics of malignant melanoma different from normal or benign tissue due to abnormal melanocyte differentiation, and quantified malignant melanoma diagnostic indicators can be provided.

Compared to the related art which can be used and interpreted only by a specialist having a great deal of clinical experience, according to an exemplary embodiment of the present disclosure, nevus and malignant melanoma can be distinguished due to mechanical characteristics that malignant melanoma tissue staining sample is measured with a scanning probe microscope.

The exemplary embodiments of the present disclosure described above can be implemented not through the apparatus and the method and can be implemented through a program which realizes a function corresponding to a configuration of the exemplary embodiments of the present disclosure or a recording medium having the program recorded therein.

While this invention has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

**Claims**

1. A method for determining malignant melanoma by a scanning probe microscope system with a cantilever, the method comprising:

   setting locations of a plurality of measurement points to be measured in sample tissue;
   applying force in a predetermined range to each measurement point on the sample tissue through the cantilever and acquiring information on a distance between a probe and the sample tissue depending on force for each measurement point;
   generating a force-distance graph of measurement points based on distance information depending on the force acquired at the plurality of measurement points; and
   determining whether the sample tissue is malignant melanoma based on characteristics information of the sample tissue extracted from the force-distance graph.

2. The method of claim 1, wherein the characteristics information
   includes at least one of linearity of the force-distance graph, a slope of the force-distance graph, stiffness distribution of the plurality of measurement points, and a stiffness distribution probability of the plurality of measurement points.

3. The method of claim 1, further comprising:

   generating the stiffness distribution probability graph of the sample tissue based on the force-distance graph,
   wherein in the determining of whether the sample tissue is the malignant melanoma,
   when the force-distance graph has non-linearity and the stiffness distribution probability graph has multi-peaks, the sample tissue is determined as the malignant melanoma.

4. The method of claim 3, wherein:

   in the determining of whether the sample tissue is the malignant melanoma,
   when the force-distance graph has linearity and the stiffness distribution probability graph has a single peak, the sample tissue is determined as normal tissue.

5. The method of claim 3, wherein:

   in the determining of whether the sample tissue is the malignant melanoma,
   when the force-distance graph has non-linearity and the stiffness distribution probability graph has the single peak, the sample tissue is determined as nevus tissue.

6. The method of claim 1, wherein:

in the determining of whether the sample tissue is the malignant melanoma, when a first force-distance graph of the sample tissue is different from a second force-distance graph of the normal tissue and the stiffness distribution probability graph of the sample tissue derived from the first force-distance graph has a plurality of peaks, the sample tissue is determined as the malignant melanoma.

7. The method of claim 6, wherein:

in the determining of whether the sample tissue is the malignant melanoma, when the first force-distance graph is different from the second force-distance graph and the stiffness distribution probability graph of the sample tissue derived from the first force-distance graph has the single peak, the sample tissue is determined as the nevus tissue.

8. A scanning probe microscope system comprising:

a cantilever with a probe; and a controller applying force in a predetermined range to each of a plurality of measurement points on a sample tissue through the cantilever, generating a force-distance graph of measurement points based on distance information between the probe and the sample tissue depending on force for each measurement point, and determining whether the sample tissue is malignant melanoma based on characteristics information of the sample tissue extracted from the force-distance graph.

9. The scanning probe microscope system of claim 8, wherein the characteristics information includes at least one of linearity of the force-distance graph, a slope of the force-distance graph, stiffness distribution of the plurality of measurement points, and a stiffness distribution probability of the plurality of measurement points.

10. The scanning probe microscope system of claim 8, wherein the controller generates a stiffness distribution probability graph of the sample tissue based on the force-distance graph and determines the sample tissue as the malignant melanoma when the force-distance graph has non-linearity and the stiffness distribution probability graph has multi-peaks.

11. The scanning probe microscope system of claim 10, wherein the controller determines, when the force-distance graph has linearity and the stiffness distribution probability graph has a single peak, the sample tissue as normal tissue, and determines, when the force-distance graph has non-linearity and the stiffness distribution probability graph has the single peak, the sample tissue as nevus tissue.

12. The scanning probe microscope system of claim 8, wherein the controller determines, when a first force-distance graph of the sample tissue is different from a second force-distance graph of the normal tissue and the stiffness distribution probability graph of the sample tissue derived from the first force-distance graph has a plurality of peaks, the sample tissue as the malignant melanoma.

13. The scanning probe microscope system of claim 12, wherein the controller determines, when the first force-distance graph is different from the second force-distance graph and the stiffness distribution probability graph of the sample tissue derived from the first force-distance graph has the single peak, the sample tissue as the nevus tissue.

14. The scanning probe microscope system of claim 8, wherein the cantilever has a resonance frequency of 204 to 497 KHz and a spring constant of 10 to 130 N/m.

15. The scanning probe microscope system of claim 8, wherein the sample tissue is skin tissue including epidermis and dermis.

# FIG. 1

Tissue biopsy    Paraffin embedding    Block section and staining

slide storage    AFM indentation    Cantilever

(a)

Sample tissue

Melanoma    Epidermis

Melanin cell    Dermis

(b)

FIG. 2

# FIG. 3

FIG. 4

Epidermis  Dermis

Cantilever

Cantilever

(a)

(b)

(c)

Force (μN)

Force (μN)

Force (μN)

(d)

(e)

(f)

EP 3 561 522 A1

FIG. 5

(a)　　　　　　　　　(b)　　　　　　　　　(c)

FIG. 6

(a)

(b)

(c)

# FIG. 7

S110 — Set locations of plurality of measurement points to be measured in sample tissue

S120 — Acquire distance information between probe and tissue depending on force of each measurement point by applying force in predetermined range to each measurement point on sample tissue through cantilever

S130 — Generate force-distance graph and stiffness distribution probability graph of measurement points based on distance information depending on force acquired at measurement points

S140 — Does force-distance graph have non-linearity?

No → S180 Determine sample tissue as normal tissue

Yes

S150 — Does stiffness distribution probability graph have multi-peaks?

No → Determine sample tissue as nevus tissue S170

Yes

S160 — Determine sample tissue as malignant melanoma

# EP 3 561 522 A1

<table>
<tr><td colspan="2" align="center">INTERNATIONAL SEARCH REPORT</td><td>International application No.<br>**PCT/KR2017/014538**</td></tr>
</table>

**A. CLASSIFICATION OF SUBJECT MATTER**

*G01Q 30/04(2010.01)i, A61B 5/00(2006.01)i*

According to International Patent Classification (IPC) or to both national classification and IPC

**B. FIELDS SEARCHED**

Minimum documentation searched (classification system followed by classification symbols)
G01Q 30/04; G01N 21/25; G01N 33/483; G01Q 60/26; A61B 10/00; G06F 17/00; G01Q 20/00; G01Q 60/38; G01Q 30/02; A61B 5/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: melanoma, cancer, malignant , benign, scanning probe microscope, AFM, hardness, force, distance, graph and curve

**C. DOCUMENTS CONSIDERED TO BE RELEVANT**

| Category* | Citation of document, with indication, where appropriate, of the relevant passages | Relevant to claim No. |
|---|---|---|
| Y | US 2014-0007309 A1 (PLODINEC, Marija et al.) 02 January 2014<br>See paragraphs [0100]-[0109], [0136]-[0139], [0148]-[0150], [0177]-[0178]; claims 1, 14; and figures 4, 27-28. | 1-15 |
| Y | KR 10-2016-0070627 A (SAMSUNG ELECTRONICS CO., LTD.) 20 June 2016<br>See paragraphs [0068]-[0075] and figures 7-8. | 1-15 |
| A | US 2014-0123347 A1 (THE REGENTS OF THE UNIVERSITY OF CALIFORNIA) 01 May 2014<br>See paragraphs [0041]-[0066] and figures 1-4. | 1-15 |
| A | KR 10-1536693 B1 (KOREA PHOTONICS TECHNOLOGY INSTITUTE) 14 July 2015<br>See paragraphs [0039]-[0055] and figures 1-6. | 1-15 |
| A | KR 10-1015369 B1 (KOREA UNIVERSITY RESEARCH AND BUSINESS FOUNDATION) 22 February 2011<br>See pages 8-16 and figures 1-17. | 1-15 |

☐ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 26 MARCH 2018 (26.03.2018) | **26 MARCH 2018 (26.03.2018)** |

| Name and mailing address of the ISA/KR<br>Korean Intellectual Property Office<br>Government Complex-Daejeon, 189 Seonsa-ro, Daejeon 302-701, Republic of Korea<br>Facsimile No. +82-42-481-8578 | Authorized officer<br><br>Telephone No. |
|---|---|

Form PCT/ISA/210 (second sheet) (January 2015)

17

**INTERNATIONAL SEARCH REPORT**
Information on patent family members

| International application No. |
| --- |
| **PCT/KR2017/014538** |

| Patent document cited in search report | Publication date | Patent family member | Publication date |
| --- | --- | --- | --- |
| US 2014-0007309 A1 | 02/01/2014 | CN 103460039 A | 18/12/2013 |
|  |  | CN 103460039 B | 02/12/2015 |
|  |  | EP 2649444 A2 | 16/10/2013 |
|  |  | EP 2649444 B1 | 09/12/2015 |
|  |  | ES 2562008 T3 | 02/03/2016 |
|  |  | JP 05809707 B2 | 11/11/2015 |
|  |  | JP 2013-545110 A | 19/12/2013 |
|  |  | US 8756711 B2 | 17/06/2014 |
|  |  | WO 2012-076729 A2 | 14/06/2012 |
|  |  | WO 2012-076729 A3 | 09/08/2012 |
| KR 10-2016-0070627 A | 20/06/2016 | CN 105699700 A | 22/06/2016 |
|  |  | EP 3032267 A2 | 15/06/2016 |
|  |  | EP 3032267 A3 | 12/10/2016 |
|  |  | US 2016-0169938 A1 | 16/06/2016 |
| US 2014-0123347 A1 | 01/05/2014 | RU 2010151919 A | 27/06/2012 |
|  |  | US 2011-0070604 A1 | 24/03/2011 |
|  |  | US 8652798 B2 | 18/02/2014 |
|  |  | US 9678105 B2 | 13/06/2017 |
|  |  | WO 2009-142661 A1 | 26/11/2009 |
| KR 10-1536693 B1 | 14/07/2015 | KR 10-2015-0059931 A | 03/06/2015 |
| KR 10-1015369 B1 | 22/02/2011 | KR 10-2009-0010555 A | 30/01/2009 |

Form PCT/ISA/210 (patent family annex) (January 2015)